# EUROPEAN PATENT APPLICATION

(11) **EP 4 378 499 A1**
(43) Date of publication of application: **05.06.2024**
(21) Application number: 23206480.8
(22) Date of filing: 27.10.2023
(51) Int. Cl.: A61M 5/142, A61B 5/00, A61M 5/46, A61B 5/145

(54) **SYSTEM, DEVICES, AND METHODS FOR RETRACTING AN INFUSION CANNULA OR SENSING ELEMENT TO EXTEND THE LIFE THEREOF**

(30) Priority: 01.12.2022 US 202263385653 P
(71) Applicant: Insulet Corporation, Acton MA 01720 (US)
(72) Inventor: CAUSEY, James, Simi Valley (US); NAZZARO, David, Groveland (US)
(74) Representative: Peterreins Schley

(57) **Abstract**

Disclosed are systems, processes and techniques implementable in and by medical devices that utilize the subcutaneous region of the skin. In the disclosed examples, an insertion mechanism is provided that inserts a cannula or sensing element into the subcutaneous region of the skin a first distance and retracts the inserted cannula or sensing element a second distance that is smaller than the first distance to create a cavity below the inserted cannula or sensing element. The retracted cannula or sensing element remains inserted in the subcutaneous region of the skin. The cavity provides an area for the effects of a wound response to occur and thereby mitigates possibility of occlusion and/or encapsulation of the inserted cannula or sensing element.

## Description

### BACKGROUND

Drug delivery systems include a drug delivery device and an infusion cannula and/or needle positioned subcutaneously for delivering a drug. Similarly, continuous glucose monitors have a sensing element or probe positioned subcutaneously for sensing glucose levels. Occlusions or aggregations of tissue or other substances at the tip of an infusion cannula or at the sensing portion of a sensing probe, or encapsulation of the infusion or sensing probe or cannula because of the body's wound response can inhibit functionality of the drug delivery device or continuous glucose monitor. To resolve these issues, a common remedy is to relocate the drug delivery device or continuous glucose monitor to another location, which may include replacing the drug delivery device or continuous glucose monitor with a new one.

Moreover, a user may not be aware that an occlusion or encapsulation has occurred and ineffective treatment or a delay in relocating the drug delivery device or resolving the issue may result.

### BRIEF SUMMARY

The present invention is directed to a transcutaneous insertion device and to a drug delivery device as defined in the enclosed claims. This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In one exemplary aspect, an insertion device is provided. The insertion device may include a sliding member, a cannula (e.g., in the case of drug delivery) and/or a sensing probe (e.g., in the case of analyte sensing), a stop beam, a drive mechanism, and an optional needle. The sliding member may be coupled to the cannula or sensing probe and/or the needle. The cannula or sensing probe may be operable to penetrate a surface of skin, or may be inserted through the skin in conjunction with a needle. The drive mechanism may be coupled to a linkage that is operable to advance the sliding member. When the sliding member is acted upon by the drive mechanism via the linkage, the stop beam may be operable to facilitate over-travel of the sliding member past the stop beam to allow the sliding member to retract until lodged against the stop beam. Other retraction methods may be used as would be understood by one of ordinary skill in the art.

In another aspect, an insertion device is provided that includes a sliding member, a cannula and/or sensing probe, a drive mechanism, and optionally a needle. The sliding member may be coupled to the cannula, sensing probe, and/or needle, which may be operable to penetrate a surface of skin. The drive mechanism may be coupled to a linkage or directly coupled to a spring. The sliding member may be configured to over-travel a stop beam to thereby create a cavity beyond a distal end of the cannula and/or sensing probe positioned beneath the surface of the skin.

### BRIEF DESCRIPTION OF THE DRAWINGS

FIG. 1A illustrates an exemplary implementation of a medical device with a cannula (and/or sensing probe) inserted in a subcutaneous layer of skin at a first state according to the disclosed subject matter.
FIG. 1B illustrates an exemplary implementation of a medical device with a cannula (and/or sensing probe) inserted in a subcutaneous layer of skin at a second state according to the disclosed subject matter.
FIG. 1C is an enlarged view of section A from FIG. 1B.
FIG. 2A illustrates a block diagram example of an insertion mechanism usable in a medical device.
FIG. 2B illustrates a block diagram example of the insertion mechanism usable in a medical device in an exemplary first state.
FIG. 2C illustrates a block diagram example of the insertion mechanism usable in a medical device in an exemplary second state.
FIGs. 3A-3C illustrate examples of a wearable drug delivery device implementation of the techniques described herein.

### DETAILED DESCRIPTION

The following discussion describes various applications of a technique and system for minimizing the potential for a cannula or sensing element when inserted in the subcutaneous region of the skin of a user to become occluded or encapsulated due to a wound response, or more generally, a technique to extend the useful life of a cannula or sensing element. In the disclosed examples, an insertion mechanism is provided that inserts a cannula or sensing element into the subcutaneous region of the skin a first distance and retracts the inserted cannula or sensing element a second distance that is smaller than the first distance to create a cavity in the subcutaneous region of the skin below or beyond a distal end of the inserted cannula or sensing element. The retracted cannula or sensing element remains inserted in the subcutaneous region of the skin. The cavity provides an area for the effects of a wound response to occur away from the cannula or sensing element that limits the possibility of occlusion and/or encapsulation of the inserted cannula or sensing element. By limiting the possibility of occlusion and/or encapsulation of the inserted cannula or sensing element, the life of the medical device is also extended.

The mechanisms and techniques described in this disclosure reduce the possibility of occlusion and/or wound encapsulation. The disclosed mechanisms and techniques may be applicable to a cannula of a drug delivery device, a sensing element of an analyte sensor, or any other type of medical device that may insert a tubular or probe structure in the skin of a user. The following discussion is applicable to either the drug delivery device or the analyte sensor, which are generally referred to herein as "a medical device" for ease of discussion. When examples are specifically directed toward either the drug delivery device or the analyte sensor, the discussion will refer to them accordingly.

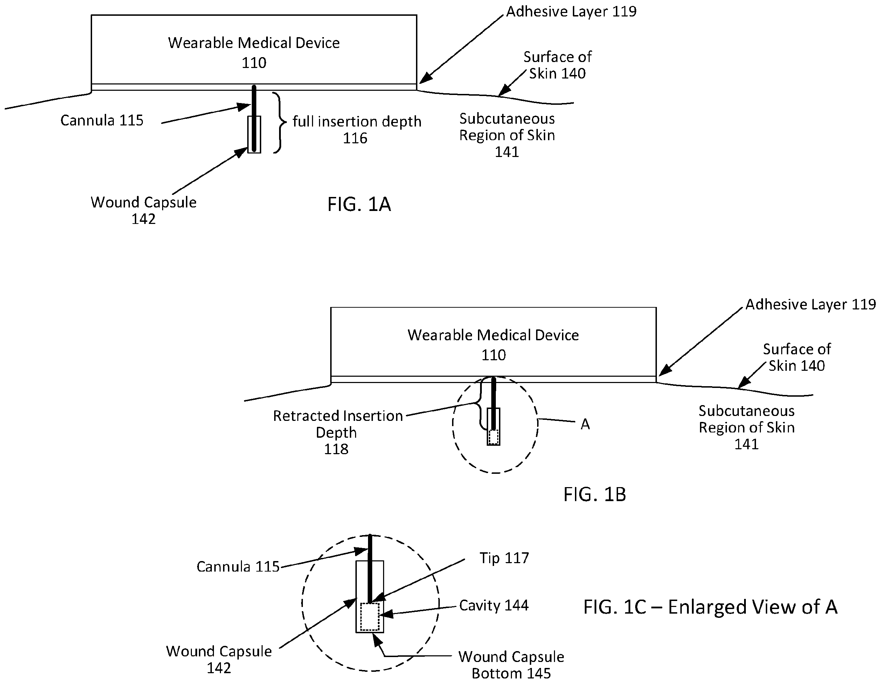

FIG. 1A illustrates an exemplary implementation of a medical device with a cannula inserted in a subcutaneous region of skin at a first insertion state. In the example of FIG. 1A, the wearable medical device 110 may include an adhesive layer 119 that is adhered to the surface of the skin 140. The wearable medical device 110 may be a drug delivery device (such as wearable insulin pump, wearable chemotherapy pump, or the like), an analyte sensor (such as a continuous glucose monitor, ketone monitor, or the like), another subcutaneous sensor or medical device (e.g., blood oxygen sensor), or the like. Alternatively, or in addition, the wearable medical device may include a needle/cannula insertion mechanism operable to insert the needle/cannula subcutaneously into a user at an infusion site and tubing to the needle/cannula insertion device located at the infusion site that is at a different location than other components of the wearable medical device. In such a configuration, the wearable medical device may be worn on a belt or carried in a bag or satchel with the tubing extending from the worn or carried wearable medical device to the needle/cannula.

In the example of FIG. 1A, the wearable medical device 110 has inserted a cannula 115 into the subcutaneous region of the skin 141. A purpose of the cannula 115 may be to deliver a liquid drug into the subcutaneous region of the skin 141. As mentioned above, the cannula 115 may be a sensing element when the wearable medical device is a sensor. For simplicity, this discussion will refer to a cannula, but the principles and embodiments are equally applicable to a sensing element of a sensor or other medical device that may be structurally different from a cannula. As shown in FIG. 1A, a first insertion state of the wearable medical device 110 may be when the cannula is at a full insertion depth 116.

When the cannula 115 of the wearable drug delivery device 110 is inserted into the subcutaneous region to the full insertion depth 116 (i.e., a first position), the cannula 115 may create a wound capsule 142. In this first insertion state, the cannula 115 extends to the bottom of the wound capsule 142. A wound response from the body may work to collapse, fill, or heal the wound capsule 142 around the cannula 115 and thereby form an occlusion or otherwise encapsulate the cannula 115. In order to either eliminate the occurrence of an occlusion and/or to prolong the life of the cannula, the wearable medical device 110 may be operable to move to a second state.

FIG. 1B illustrates an exemplary implementation of a medical device with a cannula inserted in a subcutaneous layer of skin at a second insertion state according to the disclosed subject matter. The distance from the first position to the second position may be a predetermined distance ranging e.g. from 0.2 mm to 5 mm, in particular from 0.25 to 4 mm, such as approximately 0.25 millimeters (mm), 0.5 mm, 1 mm, 1.5 mm, 2 mm, or like. To attain the second state, the wearable medical device 110 may be operable to retract a portion of the cannula from the full insertion depth 116 (i.e., the first position) to a retracted insertion depth 118 (i.e., a second position). The retracted insertion depth 118 is less than the full insertion depth 116 (where the cannula 115 is deeper into the subcutaneous region of the skin).

As shown in FIG. 1B, even when the cannula 115 is retracted, a portion of the cannula 115 remains within the subcutaneous region of the skin 141 and extends to the retracted insertion depth 118. The enlarged view of FIG. 1C shows the wound capsule 142 with the cannula 115 no longer extending to the wound capsule bottom 145. The cannula 115, in this second state, is partially retracted (or withdrawn) into the wearable medical device 110. The tip 117 of the cannula 115 is a predetermined distance away from the wound capsule bottom 145. The predetermined distance creates a cavity 144 beneath the tip 117 of the cannula 115.

By pulling the cannula 115 back toward the medical device 110 a slight distance or a portion of the original insertion depth, space in the subcutaneous tissue is provided for a capsule to form. The cavity 144 beneath the tip 117 of the cannula 115 provides space for the wound response to take place which minimizes the effects of the wound response on the cannula 115. The cavity 144 is a space for immunoglobulins, proteins, lipids and fluids that will come to the injury location to collect and mitigate the probability of an occlusion and/or encapsulation.

The wearable medical device 110 may have a structurally and functionally different cannula insertion device than an analyte sensor or another type of medical device. Different operations can take place that enable for a slight retraction of a cannula upon insertion into the subcutaneous region and the creation of a cavity below the distal tip of the cannula.

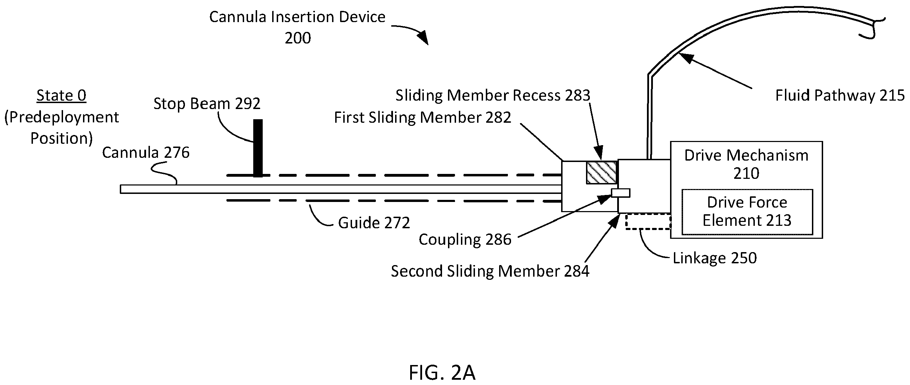

FIG. 2A illustrates a block diagram example of a cannula insertion device usable in a medical device. The block diagram of FIG. 2A shows the cannula insertion device 200 in a pre-deployment state, also referred to as a State 0. Either a wearable drug delivery device or an analyte sensor may have an insertion device 200 similar to that shown in FIG. 2A. In this example, the cannula insertion device 200 is configured to be located within a medical device, such as a wearable drug delivery device, an analyte sensor, or the like, and is operable to cause a portion of the cannula 276 to exit the medical device and penetrate the skin of the user.

In the example of FIG. 2A, the cannula insertion device 200 may include a drive mechanism 210, a linkage 250, a sliding member 282/284, a cannula 276, and a stop beam 292. In addition, the cannula insertion device 200 may include a guide 272, a fluid pathway 215, circuitry in the case of a sensing element, and/or a needle.

In the example, the sliding member 282/284 may be coupled to the cannula 276. For example, sliding members 282/284 may have a clamping structure or the like (not shown in this examples) operable to grasp or grip the cannula 276 and/or a needle. The guide 272 is configured to direct the sliding member 282/284 toward the stop beam 292. The cannula 276 in the example is operable to penetrate a surface of skin of a user. Alternatively, a needle may be used inside or outside the cannula to penetrate the skin. The sliding member 282/284 is shown as multiple components, but may be a single component. In the example of a multiple component sliding member, a first sliding member 282 may be joined to the second sliding member 284 via a coupling 286. Alternatively, no coupling 286 may be necessary. The coupling 286 between the first sliding member 282/284 may be a friction coupling, or the like. The coupling 286, if present, is operable to decouple with the application of minimal force.

As shown in the example of FIG. 2A, the sliding member 282/284 may be formed from a first sliding member 282 and a second sliding member 284. The first sliding member 282 may be configured with a sliding member recess 283 that is configured to interact with the stop beam 292. In an example in which the medical device is a wearable drug delivery device, the cannula 276 may be coupled to a fluid pathway 215 from a reservoir (not shown in this example).

The drive mechanism 210, in this example, is coupled to the linkage 250. Alternatively, drive mechanism 210 may be directly coupled to sliding member 282/284. The drive mechanism 210 includes a drive force element 213 that is operable to apply a force to the linkage. The linkage 250, in response to a force applied by the drive force element 213, is operable to advance the sliding member 282/284 in the direction of the stop beam 292. The driving force element 213 may be one or more springs, a motor, a mechanical pulley system, an elastic member, a linear actuator, or any other device that is operable to apply a force to the linkage 250. The linkage 250 may be a telescoping rod, a linked structure, a shape memory alloy, a hinged mechanism, a spring, elastic structure, or the like.

In the pre-deployment state (State 0), the cannula insertion device 200 is waiting for an actuation of the drive mechanism 210 and application of the drive force element 213. The drive mechanism 210 may, for example, be actuated by manual mechanisms, such as pushbuttons, levers, sliding elements, or the like. Alternatively, the drive mechanism 210 may be actuated by electronic circuitry and devices, such as a controller coupled to a timer, user interfaces, limit switches, or the like that may cause activation of a solenoid, a relay-like circuitry, electronic circuits, or the like. In a further alternative, actuation of the drive mechanism 210 may be by a combination of mechanisms and electronic circuitry.

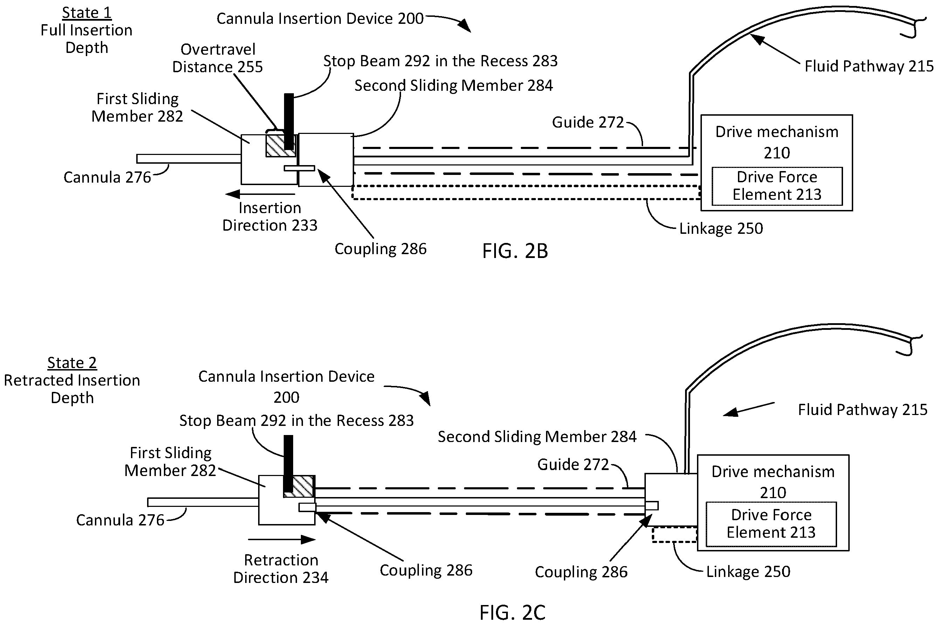

From State 0, the cannula insertion device 200 may be actuated, which causes the cannula insertion device 200 to transition to State 1 which is described with reference to FIG. 2B.

FIG. 2B illustrates a block diagram example of the insertion mechanism usable in a medical device in an exemplary first state. The block diagram of FIG. 2B shows the insertion mechanism 200 in State 1, which is the state in which the cannula 276 is inserted into the skin to a full insertion depth. It should be noted that the "full insertion depth" may not be the limit of the insertion depth of the cannula 276. For example, the full insertion depth may be a depth that is sufficient to create the cavity 144 referenced in FIG. 1B; however, in some examples, the cannula insertion device 200 may be operable to drive the cannula even deeper into the subcutaneous region of the user's skin than the full insertion depth.

In FIG. 2B, when the drive mechanism 210 actuates, the drive force element 213 may engage the linkage 250 to advance the sliding member 282/284 toward the stop beam 292. For example, the linkage 250 may cause the sliding member 282/284 to travel along guide 272 toward the stop beam 292. The linkage 250 may, for example, be a spring that expands, a structural connection, or the like, and is operable to transfer the force of the drive force element 213 to the linkage 250 to move the sliding member 282/284. In response to the force applied to the sliding member 282/284 by the linkage 250, the cannula 276 coupled to the sliding member 282/284 is operable to over-travel in the insertion direction 233 for an over-travel distance 255 past the stop beam 292. The over-travel distance 255 may be measured in tenths of millimeters, millimeters, or the like. Exemplary distances are listed above. The over-travel of the sliding member 282/284 and the cannula 276 causes the full insertion depth of the cannula 276 in the subcutaneous layer of the skin. The overtravel distance 255 may be sufficient to generate the cavity 144 shown in FIG. 1B.

In the example of a multi-component sliding member (i.e., a first sliding member and a second sliding member) shown in FIG. 2B. The first sliding member 282 is configured with an upper surface that interacts with the stop beam 292. In an example, the stop beam 292 is operable to facilitate overtravel of the sliding member 282/284 past the stop beam 292. For example, the upper surface of the sliding member 282 may be angled or substantially flat with the sliding member recess 283. The sliding member recess 283 is operable to capture the stop beam 292 and limit movement of the sliding member 282 in the insertion direction 233 but still allow for overtravel of the first sliding member 282. The second sliding member 284 is operable to apply the force of the linkage 250 to the first sliding member 282.

The sliding member recess 283 has dimensions that allow the stop beam 292 to fall into the sliding member recess 283 but still allow the sliding member to over-travel in the insertion direction 233.

Once the full insertion depth is achieved, for example, by full extension of, or substantially full extension of, the linkage 250, the linkage 250 may begin to be retracted by operation of the drive force element 213 of the drive mechanism 210 and/or the structural configuration (e.g., a spring-like action), or material configuration (e.g., an elastic-like action) of the linkage 250. The first sliding member 282 and the second sliding member 284 may be temporarily coupled together, for example, by coupling 286.

FIG. 2C illustrates a block diagram example of the insertion mechanism usable in a medical device in an exemplary second state. The second state, State 2, illustrates how the creation of the cavity 144 is completed and maintained. In the example, the first sliding member 282 and the second sliding member 284 are retracted by the linkage 250 in the retraction direction 234 toward the drive mechanism 210. The stop beam 292 is within the first sliding member recess 283, and as the first sliding member 282 and the second sliding member 284 are retracted, the first sliding member recess 282 becomes lodged against the stop beam 292 and prevents further retraction of the cannula 276.

As the linkage 250 retracts in the retraction direction 234, the coupling 286 between the first sliding member 282 and the second sliding member 284 may decouple, leaving the first sliding member 282 in place lodged against the stop beam 292. The coupling 286 may separate with a first portion of the coupling 286 in the first sliding member 282 and a second portion of the coupling 286 in the second sliding member 284. As a result of the retraction in the retraction direction 234, the cavity 144 of FIG. 1B is generated beyond the distal tip of the cannula 276 beneath the surface of the skin.

In an alternative embodiment, a needle may be attached to sliding member 284 while the cannula 276 is attached to sliding member 282. Sliding member 282 and cannula 276 may move as explained above. An optional needle may extend through cannula 276 and be retracted by sliding member 282. In this manner, a needle may be used to assist in puncturing the skin and in creating a wound capsule. Nevertheless, by allowing for cannula 115 to retract from the full insertion depth 116 to the retracted insertion depth 118, shown in FIGs. 1A and 1B, respectively, a wound capsule can be created by the cannula 115 itself rather than merely by a needle positioned within the cannula, thereby creating a larger wound capsule to prevent occlusions and/or to extend the life of the cannula 115. When a needle is used in conjunction with cannula 115, a coupling 286 may not be necessary as the frictional engagement between the needle and cannula may be sufficient to cause the cannula to retract until first sliding member 282 is caught by stop beam 292, at which time the needle may continue to retract along with sliding member 284.

In an alternative example in which the sliding member 282/284 is a single component, the linkage 250 may not fully retract to the drive mechanism 210 but may remain partially extended when the sliding member recess 283 of the sliding member 282/284 becomes lodged against the stop beam 292.

While the linkage 250 may serve to retract the cannula 276 and the sliding member 282/284 (when a single component) or first sliding member 282 (when a multiple component) as described in earlier examples, the linkage 250, in some examples, may be omitted depending upon elastic properties of the cannula 276 and/or the fluid pathway 215. In a further example, the cannula 276 may have an elastic property that enables the cannula 276 to retract and pull the sliding member 282/284 or first sliding member 282 in the retraction direction 234 to allow the sliding member recess 283 to become lodged against stop beam 292, which stops the sliding member 282/284 or first sliding member 282 from traveling farther in the retraction direction 234.

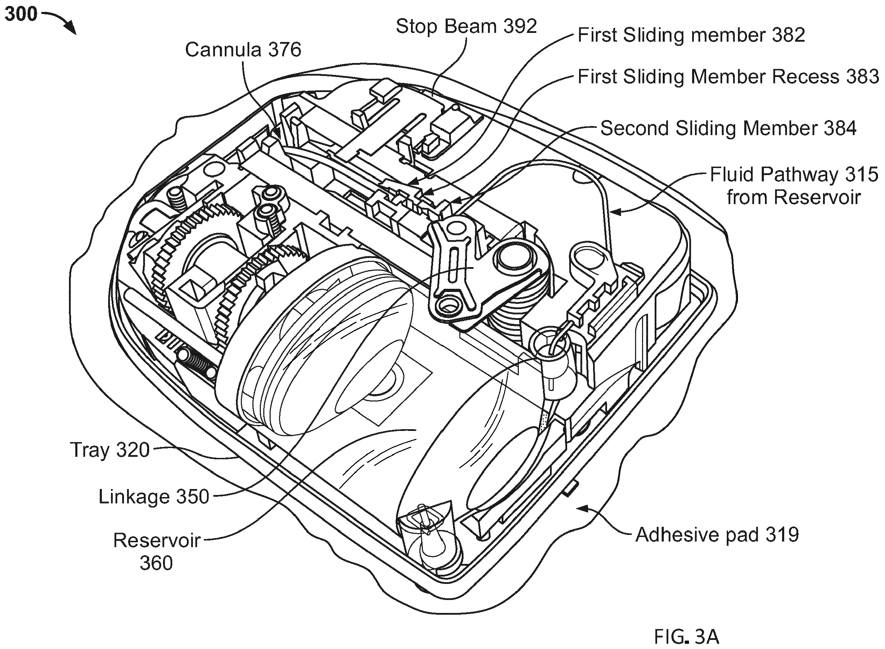

It may be beneficial to provide an example of a wearable drug delivery implementation. FIG. 3A illustrates an example of a wearable drug delivery device implementation of the described techniques in a specific example of a medical device.

In the example, the wearable drug delivery device 300 is shown equipped with a cannula insertion device that includes a cannula 376, a stop beam 392, a first sliding member 382 with a first sliding member recess 383, a second sliding member 384, a linkage 350, a fluid pathway 315, and a reservoir 360. The wearable drug delivery device 300 may fit into a housing, cradle, or tray 320 or other structure that is coupled to an adhesive pad 319. The adhesive pad 319 may be affixed to a user via an adhesive. This view of wearable drug delivery device 300 shows the device in the initial state or State 0.

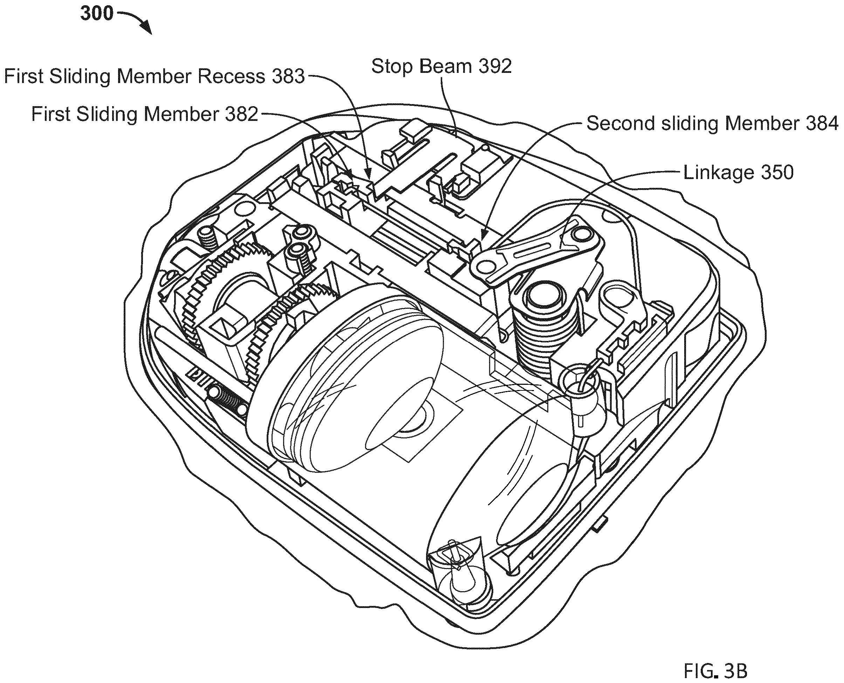

FIG. 3B illustrates an example of a wearable drug delivery device implementation of the described techniques in the specific example of a medical device in a second state.

In the view of FIG. 3B, the first sliding member 382 is shown advanced forward and latched by the stop beam 392. As shown, the stop beam 392 is lodged in the first sliding member recess 383. The second sliding member 384 is shown pulled back toward the linkage 350. In this state, State 2, the cannula 376 is retracted from the overtravel position of State 1.

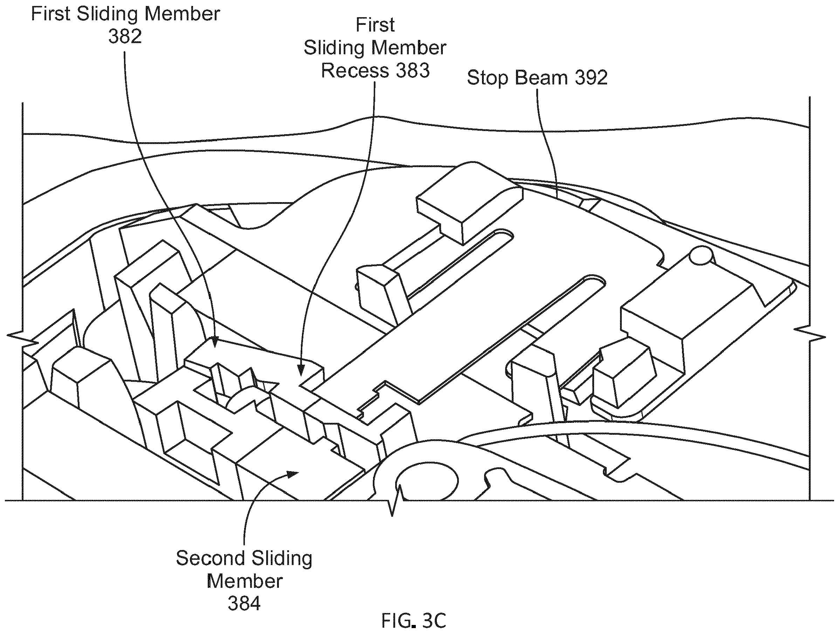

FIG. 3C illustrates a detailed view of a specific example of a stop beam and a sliding member recess. The detailed view of FIG. 3C shows the structure of the sliding member 382/384 with the first sliding member 382 having an angled surface that drops off to form the first sliding member recess 383. The stop beam 392 is shown lodged against a wall of the first sliding member recess 383. The second sliding member 384 is shown prior to being further retracted toward the drive mechanism.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, novel subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible considering this disclosure. It is intended that the scope of the present disclosure be limited not by this detailed description, but rather by the claims appended hereto. Future filed applications claiming priority to this application may claim the disclosed subject matter in a different manner and may generally include any set of one or more features as variously disclosed or otherwise demonstrated herein.

## Claims

1. A transcutaneous insertion device (200), comprising:
a sliding member (282, 284; 382, 384) coupled to an insertion member, wherein the insertion member is a drug delivery cannula (115, 276, 376) or a blood glucose sensing probe/cannula (115), and is operable to penetrate a surface of skin (140);
a drive mechanism (210) coupled to a linkage (250), wherein the linkage is operable to advance the sliding member (282, 284; 382, 384); and
a stop beam (292), wherein, when the sliding member (282, 284; 382, 384) is acted upon by the drive mechanism via the linkage, the stop beam (292) is operable to facilitate over-travel of the sliding member past the stop beam (292) and allow the sliding member to retract until lodged against the stop beam (292) to thereby cause the insertion member to retract from a first insertion position to a second insertion position.

2. The transcutaneous insertion device of claim 1, wherein, when the sliding member is lodged against the stop beam (292, 392), a cavity (144) is generated beyond a distal tip of the insertion member beneath the surface of the skin.

3. A transcutaneous insertion device (200), comprising:
a sliding member (282, 284; 382, 384) coupled to an insertion member, wherein the insertion member is operable to penetrate a surface of skin (140);
a drive mechanism (210) coupled to a linkage (250), wherein:
in a first state, the sliding member (282, 284; 382, 384) and the linkage coupled to the drive mechanism (210) have over-traveled a stop beam (292), thereby creating a cavity (144) beyond a distal tip of the insertion member beneath the surface of the skin, and
in a second state, the sliding member is lodged against the stop beam (292) after being retracted by the linkage, wherein the linkage is returned to an initial position in the second state.

4. The transcutaneous insertion device of one of the preceding claims, further comprising a fluid pathway (215) from a reservoir coupled to the insertion member and/or circuitry connecting the insertion member to a processor.

5. The transcutaneous insertion device of one of the preceding claims, wherein the sliding member further comprises a sliding member recess operable to capture the stop beam (292) and limit movement of the sliding member to allow the overtravel of the sliding member.

6. The transcutaneous insertion device of one of the preceding claims, wherein is the sliding member further comprises:
a first sliding member (282; 382) coupled to the insertion member; and
a second sliding member (284;384) coupled to the linkage, wherein the first sliding member is configured with a sliding member recess operable to capture the stop beam and limit movement of the first sliding member in the second state.

7. The transcutaneous insertion device of claim 6, wherein the sliding member further comprises a coupling that is operable to join the first sliding member to the second sliding member.

8. The transcutaneous insertion device of claim 7, wherein the coupling is further operable to decouple from the first sliding member and the second sliding member, when the drive mechanism is in the second state.

9. The transcutaneous insertion device of one of the preceding claims, wherein the stop beam is operable to facilitate over-travel of the sliding member past the stop beam and allow the sliding member to retract until lodged against the stop beam to thereby cause the insertion member to retract from a first insertion position to a second insertion position.

10. The transcutaneous insertion device of one of the preceding claims, wherein the sliding member further comprises a sliding member recess operable to capture the stop beam and limit movement of the sliding member to allow the over-travel of the sliding member.

11. The transcutaneous insertion device of one of the preceding claims, wherein the drive mechanism further comprises a drive force element, in particular at least one spring, that acts on the linkage to advance the sliding member toward the stop beam.

12. The transcutaneous insertion device of one of the preceding claims, wherein the insertion member is coupled to a fluid pathway (215, 315) from a reservoir.

13. The transcutaneous insertion device of one of the preceding claims, further comprising a guide (272) configured to direct the sliding member toward the stop beam.

14. The transcutaneous insertion device of one of the preceding claims, wherein is the drive mechanism is actuated by a manual mechanism, an electronic circuit or a combination of both.

15. A wearable drug delivery device (110; 300) comprising a reservoir (360) for storing a liquid drug, and a drive mechanism for delivering the liquid drug to a patient through the drug delivery cannula, and a transcutaneous insertion device according to one of the preceding claims.

16. An analyte sensor comprising a transcutaneous insertion device according to one of claims 1 to 14.
